# EUROPEAN PATENT APPLICATION

(11) **EP 2 907 512 A1**
(43) Date of publication of application: **19.08.2015**
(21) Application number: 14305192.8
(22) Date of filing: 14.02.2014
(51) Int. Cl.: A61K 31/165, A61K 31/42, A61K 39/395, A61P 31/12

(54) **Inhibitors of MMP-12 as antiviral Agents**

(71) Applicant: COMMISSARIAT A L'ENERGIE ATOMIQUE ET AUX ENERGIES ALTERNATIVES, 75015 Paris (FR); THE UNIVERSITY OF BRITISH COLUMBIA, Vancouver, British Columbia V6T 1Z3 (CA)
(72) Inventor: The designation of the inventor has not yet been filed
(74) Representative: Gallois, Valérie

(57) **Abstract**

The present invention relates to the use of MMP-12 inhibitors as antiviral agents, in particular MMP-12 selective inhibitors acting specifically on extracellular MMP-12.

## Description

### FIELD OF THE INVENTION

The present invention relates to the field of medicine, in particular to the antiviral treatment.

### BACKGROUND OF THE INVENTION

Interferons (IFNs) are released by host cells in response to the presence of pathogens such as viruses. They allow for communication between cells to trigger the protective defenses of the immune system that eradicate pathogens. IFN regulate innate antiviral immunity and IFNα is key in the initial response. More specifically, IFNα and IFNβ are expressed by cells in response to viral infection and communicate between cells to trigger antiviral responses.

Both IFNα and IFNβ are produced by most cells and signal via the IFNα/β receptor. Although it is known that Toll-like receptors 7, 8 and 9 activation leads to IFNα production, regulation of IFNα secretion is currently unknown. With robust and broad antiviral activity, several recombinant and pegylated forms of IFN designed for improved stability and half-life are already in clinical use to treat a multitude of viral infections. However, IFN treatment with therapeutic intent involves toxicities, such as malaise, fever, chills, fatigue and anorexia, depending on the administered dose. Therefore, potentiation of endogenous IFN levels as reported in this invention could represent a promising alternative to direct IFN administration.

### SUMMARY OF THE INVENTION

The inventors identified the surprisingly dual role that matrix metalloproteinase-12 (MMP-12, macrophage metalloelastase) has on IFNα. More specifically, extracellular MMP-12 terminates the antiviral response through clearance of secreted IFNα, whereas, on the intracellular level, MMP-12 promotes secretion of IFNα. They conclude that extracellular MMP-12 is a new target for antiviral therapy by allowing a decreased or delayed clearance of circulating IFNα.

Therefore, the present invention relates to MMP inhibition, specifically by utilizing a MMP-12 inhibitor for use as an antiviral drug or for use for treating a viral infection, and the use of a MMP-12 inhibitor for the manufacture of a medicament for treating viral infection. It further relates to a method for treating a viral infection in a subject in need thereof, comprising administering a therapeutically effective amount of a MMP-12 inhibitor. Preferably, the MMP-12 inhibitor acts specifically on extracellular MMP-12 rather than intracellular MMP-12.

Preferably, the inhibitor is selective of MMP-12, i.e. over the other MMPs. More preferably, the MMP-12 inhibitor has a low cell membrane permeability, or is a membrane-impermeable inhibitor. In particular, the MMP-12 inhibitor comprises at least one, two or three negative charges at a physiological pH or is linked to a radical bearing the negative charges, directly or by a spacer.

Preferably, the MMP-12 inhibitor has the following formula I: 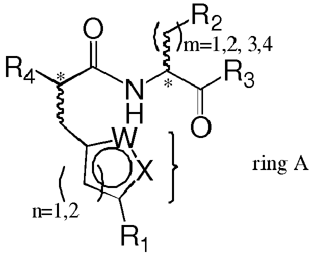 in which:
- n is 1 or 2,
- when n = 1, W and X, independently of one another, are O, N or C,
- when n = 2, W and X are C,
- R₁ is chosen from an iodine atom or a phenyl, biphenyl, 3'-chlorobiphenyl, phenoxy, phenoxymethyl, phenylethynyl, pyrimidine, 1-methyl-1 *H*-pyrazole, 5-methyl-1,2,4-oxadiazole, 1,2,3-thiadiazole, 1*H*-pyrrole, thiazole, thiophene, 3a,7a-dihydrobenzo[*d*]thiazole, 3-aminophenyl, 3-hydroxyphenyl, 3-nitrophenyl, 3-carboxyphenyl, 3-chlorophenyl, 3,5-dichlorophenyl, 3-methoxyphenyl or 3-hydroxymethylphenyl group, or a thiophene ring substituted in positions, independently of one another, 2 and/or 3 and/or 4, with a group chosen from a methyl, phenyl or 3a,7a-dihydrobenzo[*d*]thiazole group or a hydrogen atom,
   - m is an integer between 1 and 4 inclusive, and
   - when m = 1, R₂ is a carboxylic acid group or a 4-hydroxyphenyl group or a *1H-*imidazole group or a hydroxyl group or an isopropyl group or a methyl group,
   - when m = 2, R₂ is a carboxylic acid or carboxamide group,
   - when m = 3, R₂ is a carboxylic acid group,
   - when m = 4, R₂ is an amino group,
   - R₃ is chosen from an amino group; a carboxymethylpiperidine group, a carboxymethyl-3-aminophenyl group; a glutamate residue of L or D configuration, a homoglutamate residue, an aspartate residue, a glutamine residue, an alanine residue, a lysine residue, an arginine residue, a tyrosine residue, a histidine residue, a serine residue or a leucine residue, it being possible for the terminal carboxylic functions of said amino acids to be carboxamide functions -C(=O)NH₂, and said R₃ group being bonded to the carbonyl group of formula (1) via an amino function, and
- R₄ is selected from the group consisting of H, a carboxymethyl group -CH₂COOH and a benzylphosphinate, optionally substituted by Br in para..

Preferably, R₂ is a carboxylic acid or carboxamide group, R₃ is selected from the group consisting of a carboxymethyl-3-aminophenyl group, an aspartate residue, a glutamate residue, a glutamine residue, a lysine residue, an arginine residue and homoglutamate or their carboxamide derivative, and R₄ is selected from the group consisting of H, a carboxymethyl group and a benzylphosphinate, optionally substituted by Br in para.

In a first particularly preferred embodiment, the compound has the formula (I-A) and R₁ is a 3'-chlorobiphenyl group, R₂ is a carboxylic acid group and R₃ is selected from the group consisting of a carboxymethyl-3-aminophenyl group, an aspartate residue, a glutamate residue and homoglutamate or their carboxamide derivative, and R₄ is selected from the group consisting of H, a carboxymethyl group and a benzylphosphinate, optionally substituted by Br in para.

In a second particularly preferred embodiment, the compound has the formula (I-C) and R1 is a thiophene substituted in position 3 by phenyl, R₂ is a carboxylic acid or carboxamide group, R3 is selected from the group consisting of a carboxymethyl-3-aminophenyl group, an aspartate residue, a glutamate residue, a glutamine residue, a lysine residue, an arginine residue and homoglutamate or their carboxamide derivative, and R₄ is selected from the group consisting of H, a carboxymethyl group and a benzylphosphinate, optionally substituted by Br in para. More preferably, the compound has the formula (I-C) and R1 is a thiophene substituted in position 3 by phenyl, R₂ is a carboxylic acid group, R3 is selected from the group consisting of a carboxymethyl-3-aminophenyl group, an aspartate residue, a glutamate residue and homoglutamate or their carboxamide derivative, and R₄ is selected from the group consisting of H, a carboxymethyl group and a benzylphosphinate, optionally substituted by Br in para.

In a very particular embodiment, the MMP-12 inhibitor is selected from the group consisting of RXP470, and compounds 95, 95bis, 95ter, 95-4, 97, 98, 99, 101, 105 and 106, preferably selected from the group consisting of RXP470, and compounds 95, 95bis, 95ter, 97, 101 and 106.

Optionally, the MMP-12 inhibitor is used in combination with an additional drug, preferably an antiviral drug.

The viral infection can be an acute viral infection. Acute viral infections can be respiratory, enteric, or systemic, ranging from mild to severe in morbidity and mortality. They can be common, seasonal, pandemic or outbreak. For example, Respiratory Syncytial Virus, influenza, Rhinovirus, Coronavirus, Adenovirus, Bunyaviruses, Poxviruses, Paramyxoviruses like Measles, Rubella virus infection treatments may benefit from the invention, so as Poliovirus, Coxsackie virus, Adenovirus, Reoviridae (including the Norwalk rotaviruses) and other enterovirus infections, enteric or systemic.

Optionally, the viral infection can include chronic viral infections like hepatitis, in particular hepatitis B, C or D, especially chronic hepatitis C or chronic hepatitis B, herpes viruses (HSV, CMV, VZV), rotaviruses, HIV infection, laviviridae and chronic respiratory tract infections due to viral infection. Since there are so few effective antiviral drugs and given that MMP-12 is expressed in myeloid cells (macrophages, mDCs, etc) which are involved in antiviral immunity, any pathogenic viruses would be a good targets as myeloid cells are involved in the host response to the pathogenic process. Treatment may target the acute phase of a chronic viral infection to decrease viral load thereby reducing the chronic viral load/viremia set point. Treatment may target the chronic phase of virus replication to decrease the set point of chronic virus replication on its own or as a part of combination treatment.

### BRIEF DESCRIPTION OF THE DRAWINGS

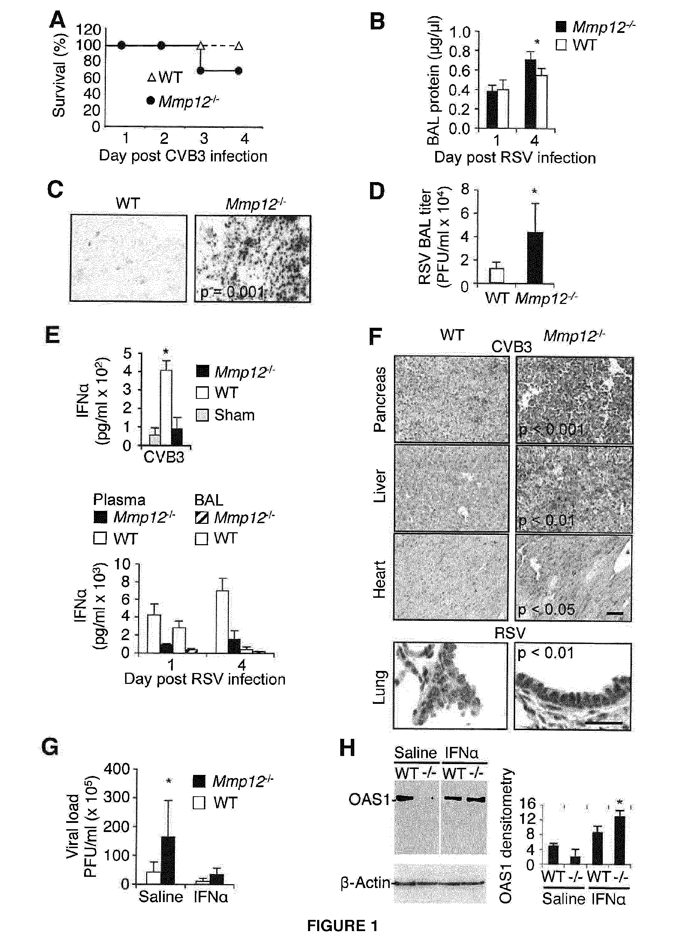

**Figure 1** **: MMP-12^{-/-} mice demonstrate increased viral load, morbidity, mortality and lower IFNα levels in viral infections. (A)** Kaplan Meier curve showing mortality rates during coxsackievirus type B3 infection. MMP-12^{-/-} (n = 16) and wild type (WT) counterparts (n = 16) were euthanized 4 days post-infection due to high mortality and morbidity in the MMP-12^{-/-} mice. (B) Bronchoalveolar lavage (BAL) was performed on MMP-12^{-/-} (n = 10) and wild-type (WT) (n = 10) mice infected with respiratory syncytial virus (RSV). Total protein concentration was determined for lavages collected at 1 and 4 days post-infection. Using ANOVA with Tukey's test of significance, MMP-12^{-/-} mice showed significantly more total protein 4 days post-infection in comparison to WT (* p <0.05). (C) Hearts were sectioned and the coxsackievirus RNA genome in the myocardium was detected by in situ hybridization (blue staining of + sense viral RNA), images were recorded and morphometric analysis conducted to determine the relative quantity of virus staining in heart tissue (n = 32, and 10 slides per mouse). Using Student's t-test of significance, MMP-12^{-/-} mice exhibited significantly higher viral titres in the heart than wild-type mice (p = 0.03). (D) Respiratory syncytial virus (RSV) titre in the BAL fluid 4 days post-infection was significantly higher in MMP-12^{-/-} mice compared to WT mice. * Student's t-test of significance p < 0.05. (E) IFNα levels in plasma and BAL samples from CVB3 and RSV infected mice were quantified using ELISA. Using ANOVA with Tukey's test of significance determined that at day 4 CVB3 infected wild-type mice (n = 16) had significantly greater plasma IFNα levels than infected MMP-12^{-/-} mice (n = 16; * p < 0.05). MMP-12^{-/-} mice (n = 10) infected with RSV had less IFNα in both their plasma and BAL samples at 1 and 4 days post-infection in comparison to infected WT mice (n = 10). (F) IFNα (brown immunostaining) in heart, pancreas and liver from CVB3 infected MMP-12^{-/-} and WT mice (n = 32, and 10 slides per mouse) and in lung from RSV infected MMP-12^{-/-} and WT mice (n = 20, and 10 slides per mouse) (scale bar = 50 µm). Hematoxylin counterstain shows nuclei in blue. Staining was quantified by morphometry using a color segmentation algorithm. Using Student's t test to compare staining morphometry values between MMP-12^{-/-} and 29 WT mice showed that there was significantly greater IFNα immunostaining was found in MMP-12^{-/-} versus WT mice in all tissues examined: pancreas p < 0.001; liver p < 0.01; heart p < 0.05; lung p < 0.01. (G) MMP-12^{-/-} (n = 24) and WT mice (n = 24) were injected with IFNα to determine if the antiviral response was intact in MMP-12^{-/-} mice. Mouse IFNα was administered via the tail vein of MMP-12^{-/-} mice, which were then infected with CVB3. Three days later mice were sacrificed. Western blotting demonstrates reduction of oligoadenylate synthase (OAS-1) in the hearts of MMP-12^{-/-} mice compared to the hearts of WT mice treated with saline, but with significant enhancement of OAS-1 upon treatment of MMP-12^{-/-} mice with recombinant mouse IFNα. β-actin is a loading control. (H) Plaque assay and (I) quantitative morphometry of in situ hybridization were conducted to determine viral load in the hearts of WT (n = 24) and MMP-12^{-/-} (n = 24) mice. ANOVA with Tukey's test of significance on the plaque assay in (H) and morphometry of the in situ hybridization in (I) determined that IFNα treatment reduced cardiac viral load in the MMP-12^{-/-} mice to WT levels indicating that a lack of systemic IFNα was responsible for their antiviral deficiency.

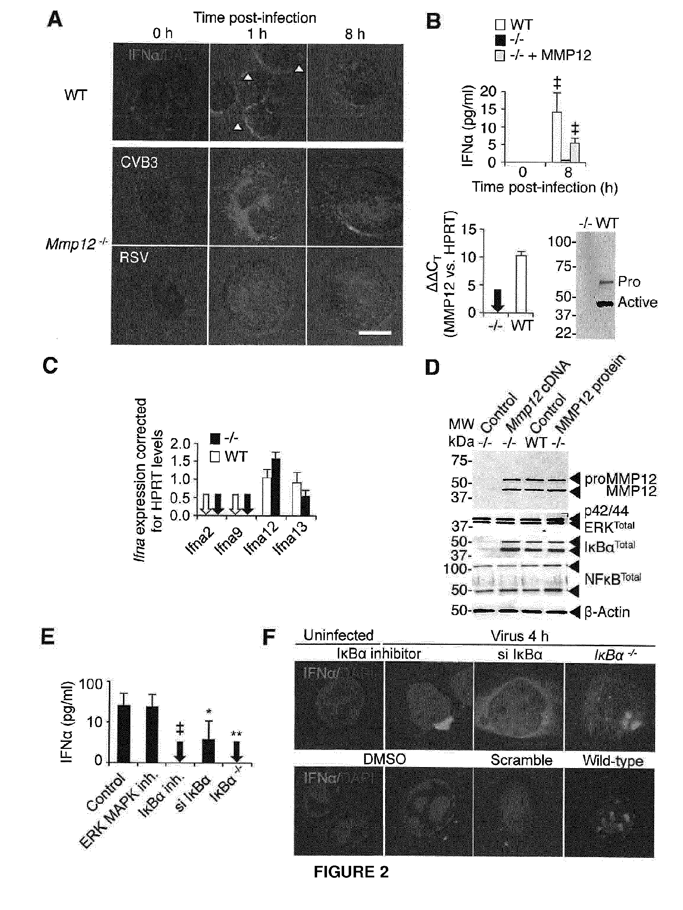

**Figure 2** **: IFNα is synthesized, but not secreted from MMP-12^{-/-} cells due to IκBα deficiency. (A)** IFNα was synthesized and sequestered in MMP-12^{-/-} fibroblasts infected with coxsackievirus type B3 (CVB3) and respiratory syncytial virus (RSV) (scale bar = 10 µm). Red color shows extensive cytoplasmic localization of IFNα in MMP-12^{-/-} fibroblasts at early and late time points postinfection. Minimal cytoplasmic IFNα was apparent in wild-type cells where some protein was located in small punctate regions at the cell periphery (arrowheads) prior to secretion. (B) IFNα secretion was quantified by ELISA of conditioned media from CVB3-infected wild-type (WT), MMP-12^{-/-} and MMP-12^{-/-} fibroblasts transfected to express murine MMP12 (^{-/-} + MMP12), which rescued IFNα secretion in the knockout cells. Significance was determined by ANOVA with Tukey's test of significance: ‡ p <0.001. Lower panel, fibroblasts and mRNA thereof were isolated from wild type and MMP-12^{-/-} 30 mice and qRT-PCR conducted. MMP-12 mRNA levels were normalized to hypoxanthine-guanine phospho-ribosyl-transferase (HPRT), a housekeeping gene standard. Error bars denote standard deviation of the mean. Western blotting for pro and active MMP-12 confirmed the absence of MMP-12 in the null cells. (C) MMP-12^{-/-} and wild-type (WT) murine embryonic fibroblasts were infected with respiratory syncytial virus and the mRNA levels for IFNα genes IFNa2, 9, 12, and 13 were determined at 4 h by qRT-PCR and normalized to HPRT. Error bars denote standard deviation of the mean. There was no significant difference in expression of INFa12 and INFa13 in MMP-12^{-/-} versus wild-type cells indicating a post-transcriptional defect in IFNα expression in MMP-12^{-/-} cells. (D) Western blot analysis for MMP-12 and candidate regulators of IFNα expression in mouse fibroblasts 4 h post-CVB3 infection. MMP-12, ERK p42/p44 MAP kinase, IκBα, NFκBα and **β**-actin were detected by Western blotting. The concentrations of all proteins are similar except IκBα concentration was virtually absent in MMP-12^{-/-} cells. IκBα was rescued by MMP-12 transfection (cDNA) or addition of recombinant MMP-12 protein to MMP-12^{-/-} cultures 16 h prior to harvest for Western blotting. (E) To confirm the role of IκBα in regulating IFNα secretion, HeLa cells were pre-treated with IκBα inhibitor Bay11-7082, ERK MAP kinase inhibitor U0126, or DMSO (control), and then infected with CVB3. IFNα was secreted from the DMSO and U0126 treated HeLa cells as quantified by ELISA, but not by the Bay11-7082 (IκBα-inhib) treated cells. Knockout IκBα^{-/-} NIH 3T3 cells and HeLa cells partially silenced for IκBα expression using RNA interference (si IκBα) also did not secrete IFNα to the medium. Significance was determined by ANOVA with Tukey's test of significance: * p < 0.05; **p < 0.01; t p < 0.001. (F) HeLa cells (wild-type) were infected with CVB3 for 4 h, fixed and immunostained for IFNα (green) and nuclei (blue). Cells were treated as follows: with Bay11-7082 (IκBα inhibitor) or DMSO control, and transfected with si IκBα oligonucleotides or scrambled controls. **IκBα^{-/-}** NIH 3T3 confirmed the essential role for **IκBα** in **IFNα** secretion. Note that inhibition of **IκBα** phosphorylation with Bay11-7082 or reduced IκBα expression in **IκBα^{-/-}** 31 NIH 3T3 or **IκBα** silenced cells (si IκBα) demonstrated greater intracellular **IFNα** staining (green) signifying retention of IFNα during virus infection.

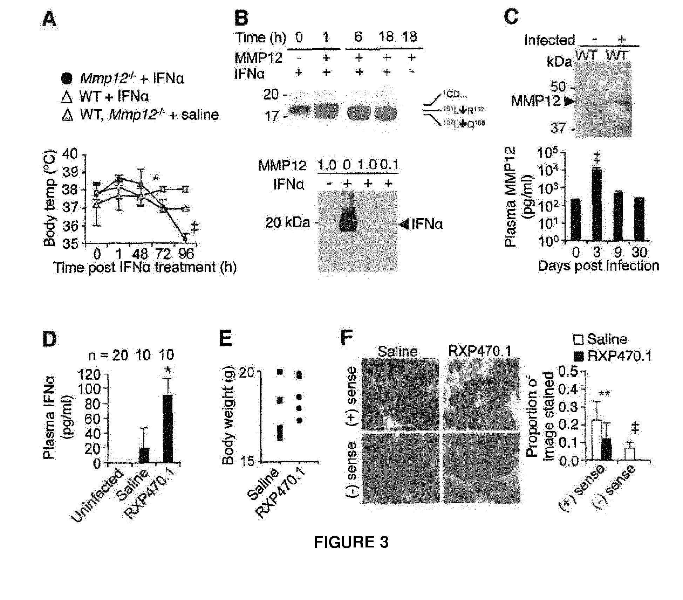

**Figure 3** **: Targeted drug inhibition of extracellular MMP-12 during virus infection enhances plasma levels of IFNα, significantly reducing viral load and morbidity. (A)** Murine IFNα was administered via the tail vein of wild type (WT) and MMP-12^{-/-} mice and infected with coxsackievirus type B3 for 4 days. Effective systemic levels of IFNα were reached in the injected mice as shown by elevation of temperature measured intra-rectally 1 h post-IFNα delivery. Unlike wild type and saline-treated mice, which demonstrated no difference in temperature, IFNα treated MMP-12^{-/-} mice experienced a profound drop in body temperature at 96 h, demonstrating loss of physiological equilibrium due to profound dysregulation of IFNα (n = 20). Standard deviation of the mean is shown as error bars. Significance was determined by ANOVA with Tukey's test of significance: * p < 0.05; t p < 0.001. (B) In vitro cleavage of IFNα-2A by MMP-12 (1:50 enzyme:substrate, 1-18 h) shows two C-terminal cleavages that were efficiently generated in under 1 h. Cleavage sites were determined by MALDI-TOF MS and Edman degradation (Supplementary Fig. 10a). The full electrophoretic gel of the lanes shown here and the entire time course is presented uncut in Supplementary Fig. 10b. The Western blot below shows that with increasing MMP-12 concentration that IFNα degradation is complete at 18 h. (C) Plasma levels of MMP-12 increase 100-fold during coxsackievirus type B3 infection. MMP-12 was detected by western blotting at day 3 of infection and quantified during infection up to 30 days by ELISA (below). Significance was determined by ANOVA with Tukey's test of significance: t p < 0.001. (D) A/J mice infected with coxsackievirus type B3 (n = 20) were treated with 100 nM of the specific MMP-12 inhibitor RXP470.1 (n = 10) or saline (n = 10) by continuous mini-pump 36 infusion for 7 days. ELISA showing RXP470.1 significantly enhanced plasma IFNα levels after 96 h virus infection by reducing the normal clearance of systemic IFNα by MMP-12. Uninfected control animals (n = 20). Significance was determined by ANOVA with Tukey's test of significance: * p = 0.05. (E) Specific extracellular MMP12 inhibition reduces morbidity and preserves body weight in coxsackievirus type B3 infection. The difference in body weight between the treatment groups (n = 20) is statistically significant at p = 0.04, whereas there was no weight difference between the groups prior to coxsackievirus B3 infection. (F) Coxsackievirus replication was detected by in situ hybridization of the viral RNA genome (+ sense stained blue) and replication intermediates (- sense) in pancreas from infected mice. Morphometric quantification (n = 20 mice, 10 slides per mouse) shows less virus in the RXP470.1-treated mice. Significance was determined by ANOVA with Tukey's test of significance: ** p < 0.01; ‡ p <0.001.

### DETAILED DESCRIPTION OF THE INVENTION

The present invention is based on the surprising identification of the surprising dual role of MMP-12 in the IFNα regulation. Indeed, while the inventors proved that intracellular MMP-12 is necessary for IFNα secretion, extracellular MMP-12 is involved in the clearance of circulating IFNα. Therefore, by inhibiting the activity of the extracellular MMP-12, it is possible to decrease the clearance of circulating IFNα, and thereby to increase the amount of IFNα while not impacting its intracellular effect. Then, MMP-12 inhibitors, through their effect on circulating IFNα, have an antiviral effect.

### MMP-12 INHIBITORS

The MMP-12, a matrix metalloprotease 12, is also called macrophage elastase (UniProtKB ID: P39900; Reference amino acid sequence: NP_002417.2; Reference mRNA: NM_002426.4).

By inhibitor is intended a molecule which decreases the MMP-12 activity, in particular its protease activity, by at least 50, 60, 70 80, 85, 90, 95, or 100 % in comparison by the activity of MMP-12 in absence of the inhibitor. In particular, the inhibitory activity is assessed by the inhibition constant, Ki. Ki can be determined by the method disclosed in Devel et al (2006, J Biol Chem, 281, 11152-11160, see section "Enzyme Assays and inhibition Studies" p 11153-4), the disclosure of which is incorporated herein by reference. In the present invention, any known MMP-12 inhibitor can be used, despite inhibitors being selective of MMP-12 over the other MMPs being preferred. The selectivity is defined preferably by comparing the Ki for MMP-12 to Ki for other MMPs. Preferably, a selective inhibitor of MMP-12 is preferably intended to refer to an inhibitor which has a Ki with at least two to three orders of magnitude less powerful toward other MMPs, an most preferably MMPs 1, 2, 3, 7, 8, 9, 11, 13 and 14 in comparison to MMP-12. In a particular embodiment, the MMP-12 inhibitor has a specificity profile for MMP-12 over other MMPs equal to or greater than RXP470.

Optionally, MMP-12 inhibitors could be antibody and derivative thereof capable of blocking the activity of MMP-12. For instance, such antibody derivatives are disclosed in WO09111507, the disclosure thereof being incorporated herein by reference. More specifically, the antibody DX-2712, disclosed in this patent application, has been shown to be potent and highly selective of MMP-12.

As used in the present invention, the term "antibody" includes any antibody or antibody-like molecule that has an antigen binding region and in particular monoclonal antibodies, chimeric antibodies, humanized or human antibodies, recombinant antibodies and fragments or derivatives thereof. Antibody fragment means, for example, F(ab)2, Fab, Fab', single domain antibodies (DABs), or sFv fragments.

Other MMP-12 inhibitors are disclosed in the following patent applications: WO10007027, WO08024784, WO06004532, WO05003115, WO05003114, US6897237, WO9804287, WO06013193, WO05019194, US2011/0281891, WO2012/038942, the disclosure thereof being incorporated herein by reference.

Selective MMP-12 inhibitors are preferred in order to avoid any side effect and to have a targeted pharmaceutical effect. Numerous selective MMP12 inhibitors have been described and are well-known by the one skilled in the art. In particular, selective MMP12 inhibitors (over other matrix metalloproteases, MMPs) are disclosed in the following patent applications: US20100227859 ; WO09007747, WO08065393, WO07068474, WO06023562, WO04020415, WO03040098, WO02074748, WO02074749, WO02074751, WO02074752, WO02074767, US7666892, US7754750, US8153673, WO0040600, US6770640, US6352976, , the disclosure thereof being incorporated herein by reference.

In addition, the selective MMP12 inhibitor can be for instance AS111793 (also called V85546 from Vernalis), commercially available at Enzo Life Sciences (ref PF-356233).

Preferably, MMP-12 inhibitors act specifically on extracellular MMP-12. By "acting specifically on extracellular MMP-12" is intended that the inhibitory effect of the MMP-12 inhibitor is mainly on the extracellular MMP-12, especially in regard to the intracellular MMP-12. In particular, the effect of the MMP-12 inhibitor on the intracellular MMP-12 is preferably minor, e.g. less than 10 or 20 % of intracellular MMP-12 inhibition in comparison with the intracellular MMP-12 activity in absence of the MMP-12 inhibitor. Alternatively, the inhibitory activities on extracellular MMP-12 and intracellular MMP-12 respectfully can be defined by a ratio. For instance, the inhibition of the extracellular MMP-12 activity is at least 5, 10, 20, 30 or 50 fold the inhibition of the intracellular MMP-12 activity.

For instance, MMP-12 inhibitors can be used at a dose which leads to an inhibitory effect mainly on the extracellular MMP-12.

Alternatively, the MMP-12 inhibitor may have physicochemical properties so as it limits the capacity of the MMP-12 to enter into cells. According to the present invention, an inhibitor having a low cell membrane permeability is an inhibitor which has a cell membrane permeability of less than 10 %, preferably 5, 3, 2 or 1 %, the % of cell membrane permeability meaning the amount of inhibitor found into cells compared to the amount of inhibitor found outside of cells when the inhibitor is incubated with cells. In a particular embodiment, the MMP-12 inhibitor is a membrane-impermeable inhibitor.

In particular, the MMP-12 inhibitor can be such that it bears negative charge(s), preferably at least one, two or three negative charges. Optionally, the negative charges can be added to a MMP-12 inhibitor, directly or through a linker, for instance a polyethyleneglycol. Alternatively, any means known to decrease cell permeability can be used to render a MMP-12 inhibitor cell membrane impermeable or low-permeable.

In a particular embodiment, the MMP-12 inhibitor has cell permeability equal to or lower than RXP470.

Preferably, MMP-12 inhibitors are selected from those disclosed in WO08/057254, WO2011/023864, Devel et al (2010, J Biol Chem, 285, 35900-35909) or Devel et al (2006, J Biol Chem, 281, 11152-11160), the disclosure thereof being incorporated herein by reference. Preferred compounds of WO2011/023864 are detailed below, their disclosure thereof being incorporated herein by reference. In particular but non-exhaustively, preferred MMP-12 inhibitors can be selected in the group comprising

### - Marimastat

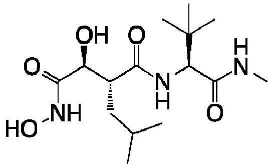

- GM6001 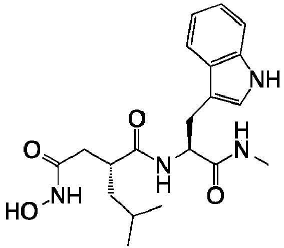 - MMP408 (CAS 1258003-93-8) with the following structure 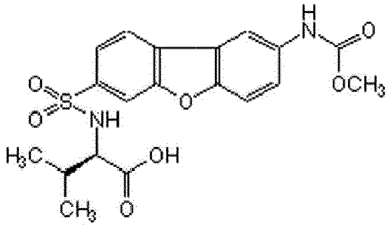
- AS111793 (2-hydroxy-3-[1-(thiophenyloxadiazolyl)-2,2-dimethylpropylcarbamoyl]-methylhexanohydroxamic acid)
- AZ11557272 (Churg et al, Thorax 2007, 62, 706-713)
- AS112108;
- AZD1236

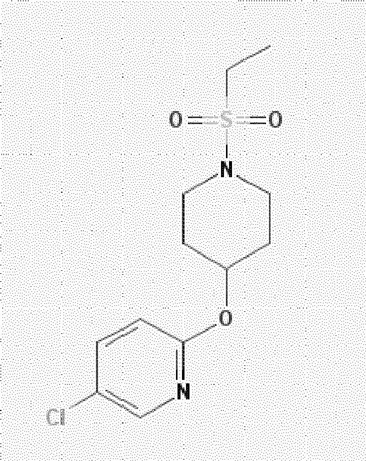

The preferred selective MMP-12 inhibitors have the following formula I: 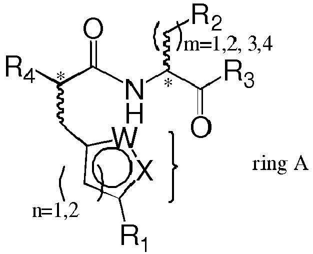 in which:
- n is 1 or 2,
- when n = 1, W and X, independently of one another, are O, N or C,
- when n = 2, W and X are C,
- R₁ is chosen from an iodine atom or a phenyl, biphenyl, 3'-chlorobiphenyl, phenoxy, phenoxymethyl, phenylethynyl, pyrimidine, 1-methyl-1 *H*-pyrazole, 5-methyl-1,2,4-oxadiazole, 1,2,3-thiadiazole, 1*H*-pyrrole, thiazole, thiophene, 3a,7a-dihydrobenzo[*d*]thiazole, 3-aminophenyl, 3-hydroxyphenyl, 3-nitrophenyl, 3-carboxyphenyl, 3-chlorophenyl, 3,5-dichlorophenyl, 3-methoxyphenyl or 3-hydroxymethylphenyl group, or a thiophene ring substituted in positions, independently of one another, 2 and/or 3 and/or 4, with a group chosen from a methyl, phenyl or 3a,7a-dihydrobenzo[*d*]thiazole group or a hydrogen atom,
   - m is an integer between 1 and 4 inclusive, and
   - when m = 1, R₂ is a carboxylic acid group or a 4-hydroxyphenyl group or a *1H-*imidazole group or a hydroxyl group or an isopropyl group or a methyl group,
   - when m = 2, R₂ is a carboxylic acid or carboxamide group,
   - when m = 3, R₂ is a carboxylic acid group,
   - when m = 4, R₂ is an amino group,
   - R₃ is chosen from an amino group; a carboxymethylpiperidine group, a carboxymethyl-3-aminophenyl group; a glutamate residue of L or D configuration, a homoglutamate residue, an aspartate residue, a glutamine residue, an alanine residue, a lysine residue, an arginine residue, a tyrosine residue, a histidine residue, a serine residue or a leucine residue, it being possible for the terminal carboxylic functions of said amino acids to be carboxamide functions -C(=O)NH₂, and said R₃ group being bonded to the carbonyl group of formula (1) via an amino function, and
   - R₄ is selected from the group consisting of H, a carboxymethyl group - CH₂COOH and a benzylphosphinate, optionally substituted by Br in para.
and the diastereoisomers and enantiomers thereof.

Preferably R₂ and R₃ are chosen in order to obtain negatively charged compounds. More preferably, R₂ is a carboxylic acid group. More preferably, R3 is selected from the group consisting of a carboxymethyl-3-aminophenyl group, an aspartate residue, a glutamate residue and homoglutamate or their carboxamide derivative for the terminal carboxylic function. More preferably, R₂ is a carboxylic acid group and R3 is selected from the group consisting of a carboxymethyl-3-aminophenyl group, an aspartate residue, a glutamate residue and homoglutamate or their carboxamide derivative. In a most preferred embodiment, R₂ is a carboxylic acid group and R3 is selected from the group consisting of a carboxymethyl-3-aminophenyl group and the carboxamide derivative of glutamate.

In one particular embodiment, R4 is a benzylphosphinate, preferably substituted by Br in position para.

Preferably, the compound may have one of the following formulae:

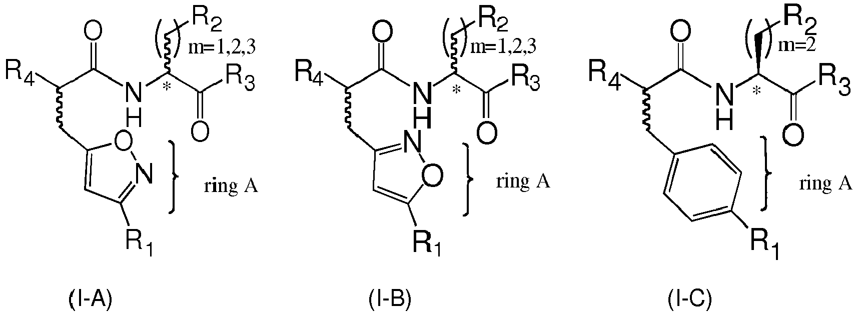

When the compound has the formula (I-A), R₁ is preferably a phenyl, biphenyl or 3'-chlorobiphenyl group. More preferably, R₁ is a 3'-chlorobiphenyl group.

When the compound has the formula (I-B), R₁ is preferably a phenyl.

When the compound has the formula (I-C), R₁ is preferably chosen from an iodine atom or a phenyl, biphenyl, 3'-chlorobiphenyl, phenoxy, phenoxymethyl, phenylethynyl, pyrimidine, 1-methyl-1*H-*pyrazole, 5-methyl-1,2,4-oxadiazole, 1,2,3-thiadiazole, 1*H*-pyrrole, thiazole, thiophene and 3a,7a-dihydrobenzo[*d*]thiazole group. When R1 is a phenyl, it can be substituted in meta by an amino, a hydroxyl, a methoxy, a nitro, a hydroxymethyl, a carboxyl or a Cl, or can have two substitutions by Cl in meta. When R1 is a thiophene, it can be substituted in position 2 by a methyl, a phenyl, or a 3a,7a-dihydrobenzo[*d*] thiazole, or in position 3 by methyl or phenyl. In a preferred embodiment, R1 is a thiophene and is substituted in position 3 by phenyl.

Optionally, R₁ is selected among the following group:

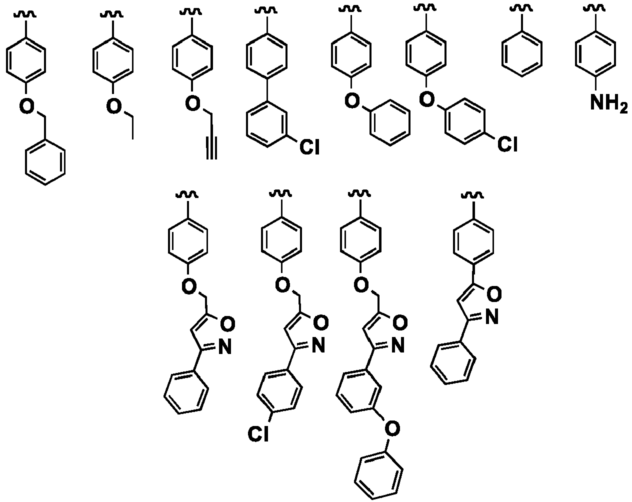

In a first preferred embodiment, the compound has the formula (I-A) and R₁ is a 3'-chlorobiphenyl group, R₂ is a carboxylic acid group and R3 is selected from the group consisting of a carboxymethyl-3-aminophenyl group, an aspartate residue, a glutamate residue and homoglutamate or their carboxamide derivative, and R₄ is selected from the group consisting of H, a carboxymethyl group and a benzylphosphinate, optionally substituted by Br in para. In a most preferred embodiment, R₂ is a carboxylic acid group, R3 is selected from the group consisting of a carboxymethyl-3-aminophenyl group and the carboxamide derivative of glutamate and R4 is a benzylphosphinate, optionally substituted by Br in para. Preferably, m is 2.

In a first preferred embodiment, the compound has the formula (I-C) and R1 is a thiophene substituted in position 3 by phenyl, R₂ is a carboxylic acid group or carboxamide group, R3 is selected from the group consisting of a carboxymethyl-3-aminophenyl group, an aspartate residue, a glutamate residue, a glutamine residue, a lysine residue, an arginine and homoglutamate or their carboxamide derivative, and R₄ is selected from the group consisting of H, a carboxymethyl group and a benzylphosphinate, optionally substituted by Br in para. Preferably, the compound has the formula (I-C) and R1 is a thiophene substituted in position 3 by phenyl, R₂ is a carboxylic acid group or carboxamide group, R3 is selected from the group consisting of a carboxymethyl-3-aminophenyl group, an aspartate residue, a glutamate residue, a glutamine residue, and homoglutamate or their carboxamide derivative, and R₄ is selected from the group consisting of H, a carboxymethyl group and a benzylphosphinate, optionally substituted by Br in para. More preferably, the compound has the formula (I-C) and R1 is a thiophene substituted in position 3 by phenyl, R₂ is a carboxylic acid group, R3 is selected from the group consisting of a carboxymethyl-3-aminophenyl group, an aspartate residue, a glutamate residue, and homoglutamate or their carboxamide derivative, and R₄ is selected from the group consisting of H, a carboxymethyl group and a benzylphosphinate, optionally substituted by Br in para. In a most preferred embodiment, R₂ is a carboxylic acid group, R3 is selected from the group consisting of a carboxymethyl-3-aminophenyl group and the carboxamide derivative of glutamate and , and R₄ is selected from the group consisting of H and a carboxymethyl group. Preferably, m is 2.

For instance, the MMP-12 inhibitor can be selected from the group consisting of compounds 3, 5-14, 16-22, 25, 28-39, 40, 42, 43, 45, 52-61, 64, 71, 74, 80, 83, 89-99, 100-103, 105, 106 and 107 disclosed in WO2011/023864, and their analogs having a bromobenzylphosphinate radical. More particularly, MMP-12 inhibitor can be selected from the group consisting of compounds 91, 92, 95, 97, 101, 103, 105, 106 and 95bis, and more particularly the compounds 95, 97, 101, 103, 105, 106 and 95bis. In a still preferred embodiment, MMP-12 inhibitor can be selected from the group consisting of compounds 95, 95bis, 105 and 106, preferably compounds 95, 95bis, and 106.

In a particular embodiment, the MMP-12 inhibitor is selected from the group consisting of the following compounds: 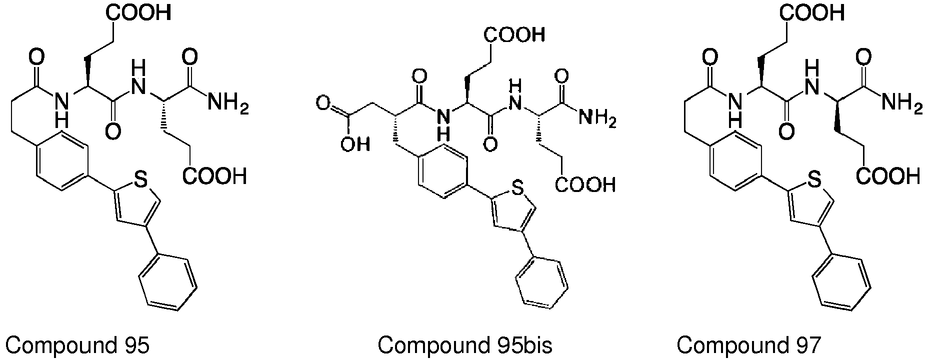 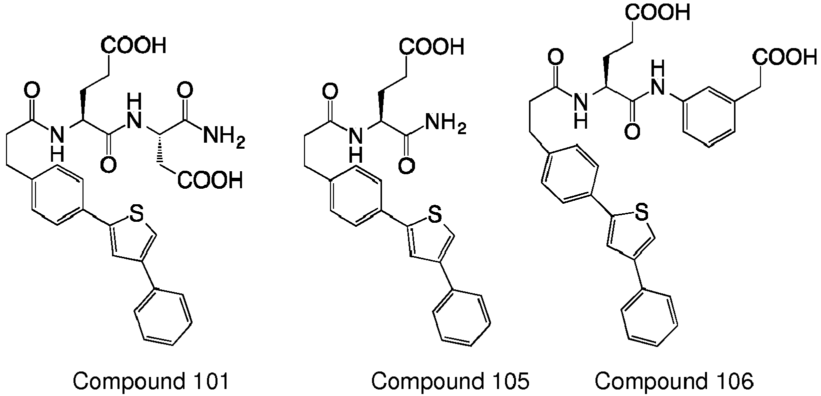 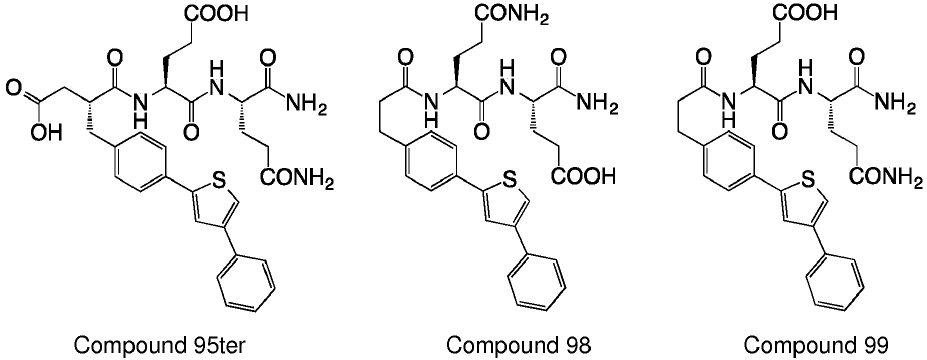 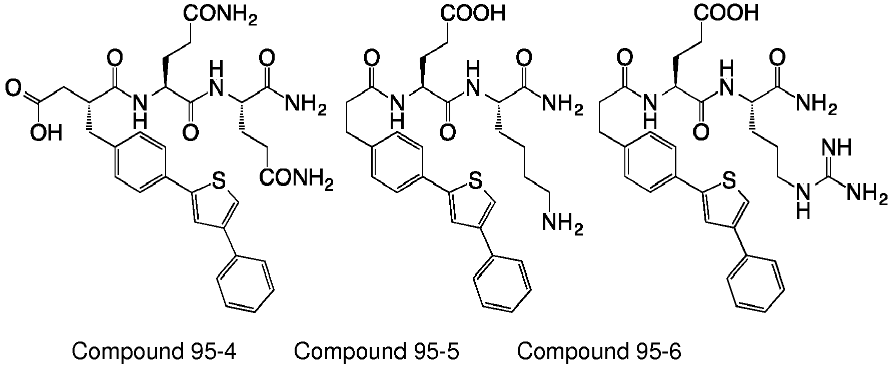 and

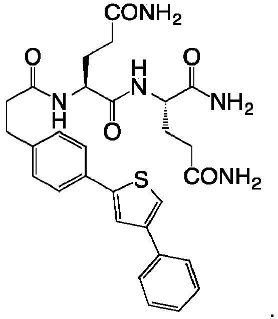

### Compound 95-7

More preferably, the MMP-12 inhibitor is selected from the group consisting of the following compounds: 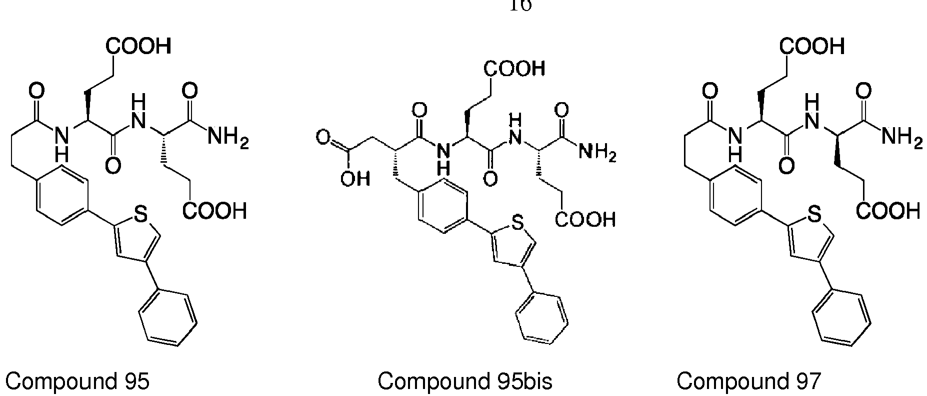 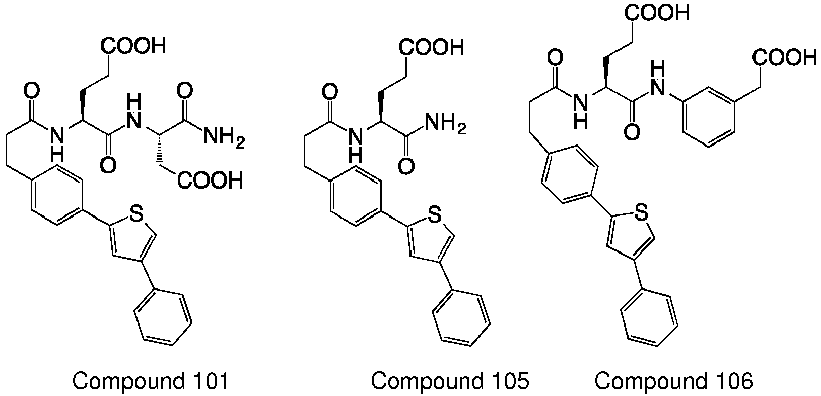 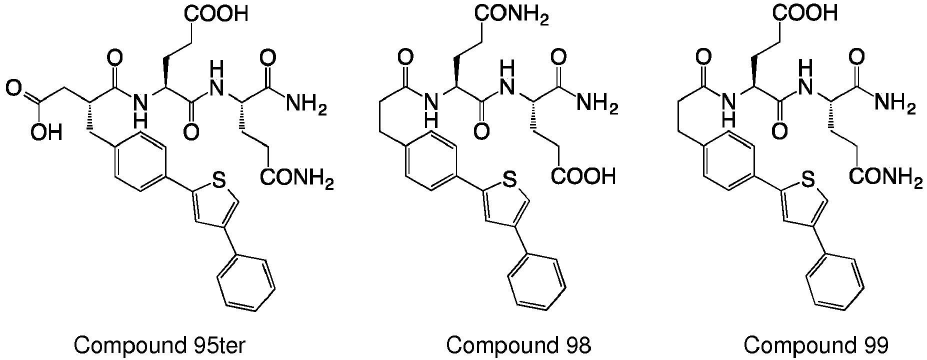 and

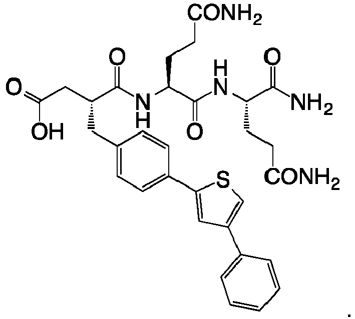

### Compound 95-4

Still more preferably, the MMP-12 inhibitor is selected from the group consisting of the following compounds: 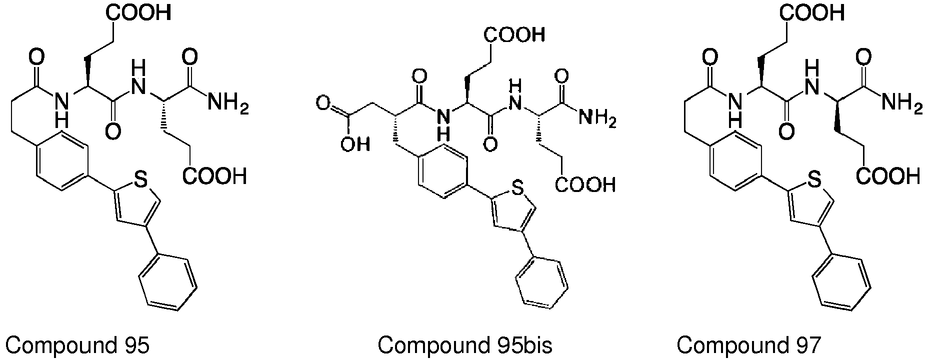 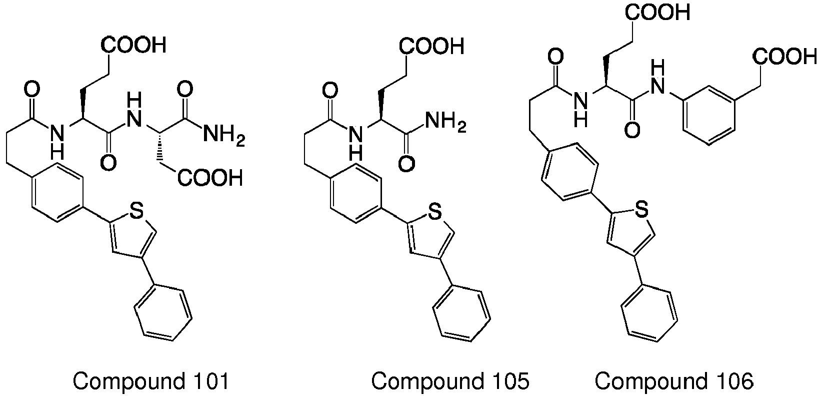 and

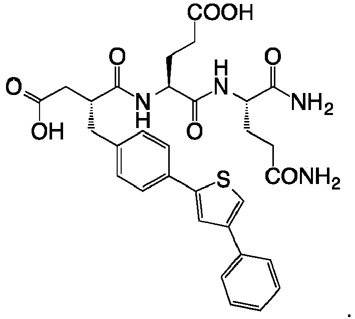

### Compound 95ter

In a particularly preferred embodiment, the MMP-12 inhibitor is RXP470.1.

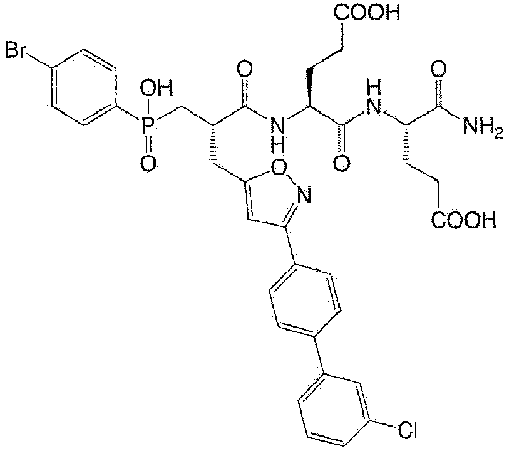

According to the present invention, MMP-12 inhibitors are useful for treating a viral infection or disease.

In a first embodiment, the viral infection or disease is an acute viral infection. Acute viral infections can be respiratory, enteric, or systemic, ranging from mild to severe in morbidity and mortality. They can be common, seasonal, pandemic or outbreak. For example, Respiratory Syncytial Virus, influenza, Rhinovirus, Coronavirus, Adenovirus, Bunyaviruses, Poxviruses, Paramyxoviruses like Measles, Rubella virus infection treatments may benefit from the invention, so as Poliovirus, Coxsackie virus, Adenovirus, Reoviridae (including the Norwalk rotaviruses) and other enterovirus infections, enteric or systemic.

In a second embodiment, the viral infection can include chronic viral infections like hepatitis, in particular hepatitis B, C or D, especially chronic hepatitis C or chronic hepatitis B, herpes viruses (HSV, CMV, VZV), rotaviruses, HIV infection, laviviridae and chronic respiratory tract infections due to viral infection.

Therefore, the present invention relates to a MMP-12 inhibitor for use as an antiviral drug or for use for treating a viral infection, and the use of a MMP-12 inhibitor for the manufacture of a medicament for treating viral infection. It further relates to a method for treating a viral infection in a subject in need thereof, comprising administering a therapeutically effective amount of a MMP-12 inhibitor.

The terms "treatment" or "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a subject and includes: (a) preventing a disease related to an undesired immune response from occurring in a subject which may be predisposed to the disease; (b) inhibiting the disease, i.e. arresting its development; or (c) relieving the disease, i.e. causing regression of the disease.

A "patient" or "subject" for the purposes of the present invention is used interchangeably and meant to include both humans and other animals, particularly mammals, and other organisms. Thus, the methods are applicable to both human therapy and veterinary applications. In the preferred embodiment the patient or subject is a mammal, and in the most preferred embodiment the patient or subject is a human. Alternatively, the patient or subject can be a pet (e.g., cats and dogs), a farm animal (e.g., cattle, sheep, chicken, swine, fish) or horses.

The term "attenuation" as used herein refers to reduction of a viral infection in a subject or in a tissue of a subject, i.e. reduction or clearance of the amount of virus or viral load. The particular degree or level of the reduction or clearance is at least 5 percent, 25 percent, 35 percent, 50 percent, 65 percent, 75 percent, 80 percent, 85 percent, 90 percent, 95 percent, 98 percent or more.

A "therapeutically effective amount" refers to that amount which provides a therapeutic effect for a given condition and administration regimen. In particular, "therapeutically effective amount" means an amount that is effective to prevent, alleviate or ameliorate symptoms of the disease or prolong the survival of the subject being treated, which may be a human or non-human animal. Determination of a therapeutically effective amount is within the skill of the person skilled in the art. The therapeutically effective amount or dosage of a compound according to this invention can vary within wide limits and may be determined in a manner known in the relevant art. The dosage can vary within wide limits and will, of course, have to be adjusted to the individual requirements in each particular case.

Examples of viral infections that may be treated by MMP-12 inhibitors include, but are not limited to, infections caused by viruses of the family Retroviridae (e.g., human immunodeficiency viruses, such as HIV-1 and HTLV;; Picornaviridae (e.g., polio viruses, hepatitis A virus; enteroviruses, human such as Coxsackie viruses, rhinoviruses, echoviruses); Calciviridae (e.g., strains that cause gastroenteritis); Togaviridae (e.g., equine encephalitis viruses, rubella viruses); Flaviviridae (e.g., dengue viruses, encephalitis viruses, yellow fever viruses); Coronaviridae (e.g., coronaviruses SARS/MARS coronaviruses); Rhabdoviridae (e.g., vesicular stomatitis viruses, rabies viruses); Filoviridae (e.g., ebola and Marburg viruses); Paramyxoviridae (e.g., parainfluenza viruses, mumps virus, measles virus, respiratory syncytial virus); Henipahviruses (Nipah and Hendra); Orthomyxoviridae (e.g., influenza viruses) or avian influenza viruses (e.g. H5N1 or related viruses); Bunyaviridae (e.g., Hantaan viruses, bunga viruses, phleboviruses and Nairo viruses); Arenaviridae

(hemorrhagic fever viruses); Reoviridae (e.g., reoviruses, orbiviurses and rotaviruses); Birnaviridae; Hepadnaviridae (Hepatitis B virus); Parvoviridae (parvoviruses); Papovaviridae (papillomaviruses, polyoma viruses); Adenoviridae (most adenoviruses); Herpesviridae (herpes simplex virus (HSV) 1 and 2, varicella zoster virus, cytomegalovirus (CMV)); Poxviridae (variola viruses, monkey pox , vaccinia viruses, other pox viruses); Iridoviridae (e.g., African swine fever virus); and unclassified viruses (, the agent of delta hepatitis (thought to be a defective satellite of hepatitis B virus), the agents of non-A, non-B hepatitis (class 1=internally transmitted; class 2=parenterally transmitted (i.e., Hepatitis C); Norwalk and related viruses, and astroviruses).

In a particular embodiment, the MMP-12 inhibitors can be used for the treatment of any viral infection or other diseases such as cancer that are known to be treated by INFα. Accordingly, the viral infection or disease can be preferably selected from the group consisting of hepatitis, in particular hepatitis B, C or D, especially chronic hepatitis C or chronic hepatitis B; and HIV infection. In addition, the MMP-12 inhibitors can be used for the treatment of respiratory tract infection due to viral infection, in particular by RSV.

In a particularly preferred embodiment, MMP-12 is used for treating an acute viral infection. Alternatively, MMP-12 is used for treating a chronic viral infection.

The MMP-12 inhibitor can be combined with an additional drug, for example as a part of combination therapy (for example: HIV triple therapy).

For instance, the additional drug can be an antiviral drug. Useful antiviral drugs that can be used in combination with MMP-12 inhibitor include, but are not limited to, protease inhibitors, nucleoside reverse transcriptase inhibitors, non-nucleoside reverse transcriptase inhibitors and nucleoside analogs. Examples of such antiviral agents include, but are not limited to, zidovudine, acyclovir, gangcyclovir, vidarabine, idoxuridine, lamivudine, emtricitabine, trifluridine, and ribavirin, as well as foscarnet, amantadine, rimantadine, saquinavir, indinavir, amprenavir, lopinavir, ritonavir, the alpha-interferons; adefovir, clevadine, entecavir, and pleconaril. In a particular preferred embodiment, the antiviral drug can be for instance ribavirin or a type I IFN.

Typical suitable interferon-alphas include, but are not limited to, recombinant IFN α-2b such as INTRON A interferon available from Schering Corporation, Kenilworth, N.J., recombinant IFNα-2a such as ROFERON® interferon available from Hoffmann-La Roche, Nutley, N.J., recombinant IFN-α 2c such as Berofor® alpha 2 interferon available from Boehringer Ingelheim Pharmaceutical, Inc., Ridgefield, Conn. IFN-α n1, a purified blend of natural alfa interferons such as SUMIFERON® available from Sumitomo, Japan or as WELLFERON® IFN-α n1 (INS) available from the Glaxo-Wellcome Ltd., London, Great Britain, or a consensus alpha interferon such as those described in U.S. Pat. Nos. 4,897,471 and 4,695,623 (especially Examples 7, 8 or 9 thereof) and the specific product available from Amgen, Inc., Newbury Park, Calif., or IFN-α n3, a mixture of natural alfa interferons made by Interferon Sciences and available from the Purdue Frederick Co., Norwalk, Conn., as ALFERON® or recombinant interferon alpha available from Frauenhoffer Institute, Germany or that is available from Green Cross, South Korea. The interferon can be in PEGylated form. A PEGylated interferon is a polyethylene glycol modified conjugate of interferon. Polyethylene-glycol-interferon alfa-2a conjugate is preferred (see EP 809 996), such as PEGASYS®. Furthermore, interferon may be fused or conjugated to a protein such as albumin.

Alternatively, the additional drug can be an anti-inflammatory agent, or an immunomodulator agent.

The present invention relates to a pharmaceutical composition comprising a MMP-12 inhibitor as defined above and optionally a pharmaceutically acceptable carrier for use for treating a viral infection. Optionally, the pharmaceutical composition may further comprise an additional drug, in particular another antiviral drug such as those as disclosed above.

The term "pharmaceutically acceptable carrier" is meant to encompass any carrier (e.g., support, substance, solvent, etc.) which does not interfere with effectiveness of the biological activity of the active ingredient(s) and that is not toxic to the host to which it is administered. For example, for parenteral administration, the active compounds(s) may be formulated in a unit dosage form for injection in vehicles such as saline, dextrose solution, serum albumin and Ringer's solution.

MMP-12 inhibitor can be administered by any suitable route, particularly by oral, parenteral, intravenously, subcutaneous, intraperitoneal, topical, transmucosal transdermal, intrabronchial, intrapulmonary and intranasal, intraventricular, intraarticular, intrathecal, intravaginal, or intratracheal administration.

The pharmaceutical composition can be formulated as solutions in pharmaceutically compatible solvents or as emulsions, suspensions or dispersions in suitable pharmaceutical solvents or vehicule, or as pills, tablets or capsules that contain solid vehicules in a way known in the art. Formulations of the present invention suitable for oral administration may be in the form of discrete units as capsules, sachets, tablets or lozenges, each containing a predetermined amount of the active ingredient; in the form of a powder or granules; in the form of a solution or a suspension in an aqueous liquid or non-aqueous liquid; or in the form of an oil-in-water emulsion or a water-in-oil emulsion. Formulations for rectal administration may be in the form of a suppository incorporating the active ingredient and carrier such as cocoa butter, or in the form of an enema. Formulations suitable for parenteral administration conveniently comprise a sterile oily or aqueous preparation of the active ingredient which is preferably isotonic with the blood of the recipient. Every such formulation can also contain other pharmaceutically compatible and nontoxic auxiliary agents, such as, e.g. stabilizers, antioxidants, binders, dyes, emulsifiers or flavouring substances. The formulations of the present invention comprise an active ingredient in association with a pharmaceutically acceptable carrier therefore and optionally other therapeutic ingredients. The carrier must be "acceptable" in the sense of being compatible with the other ingredients of the formulations and not deleterious to the recipient thereof. The pharmaceutical compositions are advantageously applied by injection or intravenous infusion of suitable sterile solutions or as oral dosage by the digestive tract. Methods for the safe and effective administration of most of these chemotherapeutic agents are known to those skilled in the art. In addition, their administration is described in the standard literature.

Further aspects and advantages of the present invention will be disclosed in the following experimental section, which should be regarded as illustrative and not limiting the scope of the present application.

### EXAMPLE

Interferonα (IFNα) is important in antiviral immunity. In this study, the inventors identified that extracellular MMP-12 terminates the antiviral response through clearance of circulating IFNα. It can be prevented by extracellular-targeted MMP-12 drugs and reduces mortality in viral infection.

### RESULTS

**Viral infection in *MMP-12*^{*-*/*-*} mice is lethal.** The inventors infected *MMP-12*^{*-*/*-*} mice with coxsackievirus type B3 and respiratory syncytial virus to determine the role of MMP-12 in viral infection. The authors infected Mmp12-/- mice withcoxsackievirus type B3, an unenveloped virus that causes systemic infection, and observed 30% death (Figure 1A) Wild-type mice did not die. Mice were also infected in a pulmonary model of respiratory syncytial virus infection, an enveloped virus that infects lung airways. As shown by elevated protein in bronchoalveolar lavage fluid, which reflects the host response to viral replication, MMP-12^{-/-} mice again showed increased morbidity compared to the wild-type mice (Figure **1B**). Although these changes were less severe when compared with coxsackievirus host responses, significantly elevated viral loads in MMP-12^{-/-} mice were observed in both coxsackievirus (Figure **1C****)** and respiratory syncytial virus (Figure 1D) infections revealing a broad antiviral deficiency linked to the absence of MMP-12.

Interferons (IFN) regulate innate antiviral immunity with IFNα being key in the initial response. In plasma from wild-type mice IFNα was elevated in response to coxsackievirus infection (Figure **1 E)****.** In contrast, in MMP-12^{-/-} mice IFNα plasma concentrations were unexpectedly low and not greater than shaminfected controls (Figure 1 E). IFNα was also markedly deficient in plasma and bronchoalveolar fluid in MMP-12^{-/-} mice at early and late time points in respiratory syncytial virus infection (Figure **1 E)****,** which is known to strongly induce IFNα due to less effective interferon evasion mechanisms. IFNα secretion is defective in MMP-12 mice. Unlike in plasma, there were surprisingly high concentrations of IFNα in tissues of coxsackievirus infected MMP-12^{-/-} mice and in the lung epithelial lining during respiratory syncytial virus infection (Figure 1 F). This supported IFNα pathway dysfunction in MMP-12^{-/-} mice as the reason for their morbidity and mortality. To determine if there was impaired IFNα responsiveness in MMP-12^{-/-} mice, we administered recombinant IFNα intravenously, which elevated cardiac levels of oligoadenylate synthase-1 (Figure **1G**), a downstream product of IFNα signalling, and decreased viral load (Figure **1H****,I**). Notably, oligoadenylate synthase-1 was low or absent in the knockout mice during viral infection prior to administration of recombinant IFNα reflecting low systemic levels of endogenous IFNα and of greatly diminished IFNα receptor activation. Hence, the antiviral defect in MMP-12^{-/-} mice lies neither in impaired capacity for IFNα cellular responses nor in synthesis of IFNα but in its secretion. Indeed, IFNα was retained intracellularly during in vitro coxsackievirus and respiratory syncytial virus infection of fibroblasts from MMP-12^{-/-}, but not wild-type mice (Figure **2A****)** and was undetectable in the culture medium of infected knockout cells (Figure **2B**). The key involvement of MMP-12 in mediating the IFNα response was confirmed by rescue of IFNα secretion following transfection of MMP-12 into the knockout cells (Figure **2B**). Western blotting and quantitative RT-PCR (Figure **2B**) confirmed this. Thus, MMP-12 was produced in fibroblasts as well as monocytes/macrophages in mice.

**IFNα expression requires IκBα, which is defective in Mmp12-/- cells :** The IFNα expression pathway is not as well understood as for other interferons. As there was no difference in IFNα gene expression in MMP-12^{-/-} versus wild-type cells in infection (Figure 2C), we assayed for defective ERK MAP kinase, NFκB or IκBα expression, which are known to be keymediators in the expression of many components of the immune response in inflammation and viral infection. There was a significantly lower level of IκBα in MMP-12^{-/-}, fibroblasts than in wild-type cells that was restored by transfection of MMP-12 or addition of MMP-12 protein (Figure **2D****).** In contrast, NFκB and ERK MAP kinase were unaltered in the MMP-12^{-/-}, compared with wild-type cells or upon MMP12 treatment (Figure 2D). Kappa-B kinase phosphorylates IκBα, initiating NFκB activation by releasing the inhibitory IκBα subunit for proteasomal degradation. NFκB then translocates to the nucleus in virus-infected cells17 where it activates more than 500 target genes involved in the immune response16. When wild-type cells were infected with coxsackievirus type B3 and treated with a chemical inhibitor of κB kinase, Bay11-7082, which decreases IκBα phosphorylation, activity, and proteasomal degradation, we found that IFNα was not secreted (Figure 2E). We confirmed this using IκBα-/- cells (Figure **2E****).** In addition, upon reducing IκBα expression by RNA interference IFNα secretion was also significantly reduced (Figure 2E) suggesting that the dynamic regulation of NFκB regulated gene transcription is altered by reduced levels of IκBα, although other inhibitor subunits of NFκB are known. Consistent with continued IFNα synthesis and a lack of secretion in these cells, IFNα staining was observed within nuclear (1 h post-infection) and perinuclear regions (4 h post-infection) (Figure 2F) as found in virus-infected MMP-12^{-/-} cells (Figure **2A****).** Thus, IFNα secretion depends on IκBα, which is deficient in MMP-12^{-/-}, cells so impairing IFNα export.

**IFNα is degraded by MMP-12:** Infusion of IFNα, which is pyrogenic, into MMP-12^{-/-} mice decreased viral load, but was associated with a significant initial elevation in MMP-12^{-/-} body temperature followed by profound hypothermia 4 days later (Figure **3A****).** The prolonged body temperature response in IFNα-treated MMP-12^{-/-} mice indicated an additional failure in attenuating IFNα-induced responses. Consistent with an in vivo role for extracellular MMP-12 in clearing systemic IFNα, MMP-12 was found to efficiently cleave the C terminus of IFNα at two sites (157Leu↓Gln and 161Leu↓Arg) as shown by MADI-TOF MS and Edman sequencing (Figure **3B**) with a kcat/K_{M} of 221 ± 14 M⁻¹ s⁻¹. Notably, the level of MMP-12 is elevated 100-fold in the plasma of wild-type mice 3 days post-infection (Figure **3C****)** and at higher enzyme concentrations IFNα is completely degraded (Figure **3B**). All 12 IFNα isoforms are susceptible to cleavage at the conserved cleavage sites. Notably, these lie in the IFNα receptor-2 binding region, the cleavage of which eliminates IFNα receptor-2 activation.

Hence, the inventors find two roles for MMP12-the first where it promotes IFNα export and a second where MMP12 later attenuates the anti-viral response in a negative feedback loop to clear systemic IFNα and so prevent prevent IFNα-toxicity.

**A MMP12 inhibitor drug reduces viral infection :** Whereas the intracellular activity of MMP-12 is beneficial for antiviral defence, we hypothesized that specifically inhibiting the extracellular activity of MMP12 in IFNα inactivation and clearance to boost systemic IFNα levels has potential for short-term broad-spectrum antiviral therapy. Moonlighting activities of drug targets are a therapeutic challenge and broad-spectrum MMP inhibitors have unacceptable side effects in man. We addressed this using an MMP-12-specific phosphinic-peptide inhibitor (RXP470.1) which, because of its double negative charge, is membrane impermeable and so was predicted to spare beneficial intracellular activities of MMP-12 (Devel et al. 2006, J Biol Chem 281, 11152-11160; Johnson et al. 2011. Arterioscler Thromb Vasc Biol 31, 528-535). We used A/J mice as these are the most susceptible to model coxsackievirus type B3 and respiratory syncytial virus infections. Wild-type mice infected with coxsackievirus type B3 virus were treated by continuous infusion of RXP470.1 using minipumps over 7 days. The wild-type mice exhibited highly elevated plasma IFNα levels (Figure 3D), consistent with reduced degradation of systemic IFNα by MMP-12, which was reflected by reduced morbidity and a significant retention of body weight compared with saline treated infected wild-type mice (Figure 3E). Notably, viral replication was near abrogated at day 7 in the drug treated wild type mice and this was reflected by an almost 50% reduced viral load. Although such a marked reduction is expected to further increase with time following continued dampening of viral replication.

Hence, MMP-12 is a new drug target candidate in acute viral infection.

### DISCUSSION

The secretion of IFNα from virus-infected cells is necessary to activate antiviral mechanisms and to alert surrounding cells to a viral infection, but the side effects of IFNα cause significant pathology implicating the presence of tight control mechanisms for IFNα. The inventors found a dual feedback control mechanism of IFNα activity by MMP-12 wherein the metalloproteinase acts early at the transcriptional level in the regulation of IκBα expression and IFNα secretion. Later, during the course of viral infection, extracellular MMP-12 degrades systemic IFNα, terminating the long term and potentially toxic effects of IFNα.

Moonlighting drug targets present therapeutic challenges, in this case where the intracellular activities of MMP-12 are protective during viral infection. However, the extracellular activities of MMP-12 offer potential for targeted drug intervention to prolong systemic levels of IFNα to enhance its antiviral actions. Currently chronic infections (hepatitis B, C and HIV) are treated with membrane-impermeable pegylated IFNα but with toxic IFNα side-effects. The present experiments with a membrane impermeable specific inhibitor that blocks only extracellular MMP-12 revealed the effectiveness of inhibiting IFNα degradation in achieving whole animal protection from viral infection. Similar membrane impermeable drugs may hold anti-viral therapeutic potential for a number of indications including acute viral infections, where few treatments exist today. Thus MMP-12 inhibition may be a broad spectrum anti-viral strategy to consider for pandemic preparedness.

### MATERIALS AND METHODS

**Mouse infection.** Five week-old male MMP-12 knockout mice (*MMP-*12^{-/-}) (n = 8) (004855) (Jackson Laboratories, Bar Harbor, ME, USA) and wild-type littermates (n = 8) were infected with coxsackievirus type B3 (Gauntt strain) (1 x 10⁵ plaque forming units (PFU) by intraperitoneal injection and followed for 4 days. In follow-up experiments, 32 *MMP-12*^{-/-} and 32 wild-type mice were infected with 10⁴ PFU to minimize morbidity and mortality as per Canadian Council for Animal Care (CCAC) guidelines. Recombinant mouse IFNα or saline vehicle was administered in the anterior tail vein of 8 *MMP-12*^{-/-} and 8 wild-type mice, repeated four times daily. Body temperature was measured using an intra-rectal digital probe (Vivo Sonics Technologies, USA) immediately after tail vein injection and 1, 48, 72 and 96 h later. Mice were treated as per the CCAC guidelines and morbidity scores were determined.

Five-week old male A/J mice (Jackson Laboratories) were infused with MMP-12 inhibitor RXP470.1 (n = 10) or saline [(0.9 %) n = 10] using Alzet 1004 osmotic minipumps (Alzet, USA). Pumps were filled and implanted subcutaneously during isofluorane anaesthesia and meloxicam analgesia, as per CCAC guidelines and manufacturer's instructions. Animals were monitored twice daily. Five days post-implantation, the mice were infected with 10⁵ PFU coxsackievirus type B3 and euthanized after 4 days. Blood was obtained via facial vein into heparin tubes and plasma was prepared for MMP-12 assay and IFNα ELISA (see below).

**Antibodies.** Polyclonal rabbit anti-oligoadenylate synthase-1 (C-terminus, AP6226a) was from AbGent, secondary antibodies conjugated with horseradish peroxidase were from Santa Cruz Biotechnology. A rabbit polyclonal antibody to IFNα (ab27715, AbCam) was used at a 1/1000 dilution on tissue sections for immunohistochemistry and for Western blotting. A goat polyclonal antibody was used to detect IFNβ (ab10740, AbCam). Six different MMP-12 monoclonal and affinity-purified polyclonal antibodies were purchased from R&D Systems, Minneapolis, MN, USA (catalytic domain, mab919), AbCam (C-terminal hemopexin domain, ab39867), and Triple Point Biologics, Forest Grove, OR, USA (pro, catalytic, hinge and hemopexin domains, antibody kit ATK-MMP-12). Anti-actin monoclonal antibody (sc7210) was from Santa Cruz Biotechnology. Antibodies for ERK MAP kinase, IκBα and NFκB (# 9102, 9242 and 3035, respectively) were from Cell Signalling Technologies.

**Recombinant proteins.** Recombinant human MMP-12 was expressed in *E*. *coli* and purified (Houghton, et al. 2006. Cancer Res 66, 6149-6155) or purchased from R&D Systems (mouse, 3467-MP-01 0; human, 917-MP-010). MMP-12 (100 ng/µl) was activated by 30 µM amino-phenyl mercuric acetate in assay buffer (50 mM Tris-HCl, 1 µM ZnCl₂, 10 mM CaCl₂, 0.15 M NaCl, 0.05 % Brij 35, pH 7.5), 37 °C. A quenched fluorescent peptide substrate (ES010) to assay MMP-12 activity was from R&D Systems (Minneapolis, USA). Recombinant mouse IFNα (HC1040b) was from HyCult Biotechnology and IFNβ (12400-1) was from R&D Systems. Human SPARCL1 (AF2728, R&D), human PSME3 recombinant protein (Novus Biologicals, H00010197-P01) and TNFα (210-TA-010/CF) was purchased from R&D.

**Inhibitors.** The MEK kinase (upstream activator of ERK MAP kinase) inhibitor U0126 (1144) and IκBα inhibitor Bay11-7083 (1743) were purchased from Tolcris pharmaceuticals.

***In situ* hybridization.** The presence of virus (- strand probe) and of active virus replication (+ strand probe) in cardiac tissue was assessed by *in situ* hybridization (Cheung, et al. 2008. Circulation 117, 1574-1582).

**Plaque assay for infectious virus in heart tissue.** Infectious virus in the hearts of coxsackievirus type B3 infected mice was determined by plaque assay (Cheung, *et al. supra).* Mouse hearts were flash frozen in liquid nitrogen, minced with mortar and pestle, reconstituted with saline and plated on HeLa indicator cells for plaque determination under agar overlay. Plaques in a limiting dilution series were counted and reported as PFU per ml.

**ELISA analysis of plasma cytokines and MMP-12.** Mouse blood was collected into heparin tubes during euthanasia and centrifuged at 14,000 xg. Bradford protein assay (BioRad) of plasma was determined. For ELISAs 100 ng total plasma protein was added to microwells. ELISA was used to measure the concentration of mouse and human IFNα (Quantikine, R&D Systems), IL-1β, IFNβ and IFNγ (Invitrogen-BioSource) and MMP-12 (Kamiya Pharmaceuticals).

**MMP-12 cleavage assays.** Recombinant IFNα and IFNβ (10²-10⁵ IU/ml) and 100 *µ*M quenched fluorescent synthetic peptide (ES01 0, R&D) were incubated with 10 or 100 ng MMP-12, 37 °C in assay buffer. The ES010 peptide has the sequence Mca-K-P-L-G-L-Dpa-A-R-NH₂ (Mca, 7-Methoxycoumarin-4-yl)acetyl; Dpa, N-3-(2,4-Dinitrophenyl)-L-2,3 diaminopropionyl). Fluorometric substrate readings were obtained at 30 min intervals (excitation 420 nm, emission 320 nm) and the assay stopped at 3 h when MMP-12 activity plateaued. IFN cleavage was determined by Western blot. MMP-12 activity in plasma from mice treated with MMP-12 inhibitor RXP470.1 or saline vehicle control was determined using the Sensolyte 520 MMP-12 activity assay (Anaspec).

**Statistical analysis.** Standard deviation and analysis of variance were used to analyse data variability: The Student's *t*-test was used to determine statistical significance between 2 treatments, and Tukey's family comparison was used for >2 treatments. A P value <0.05 was considered statistically significant.

## Claims

1. A MMP-12 inhibitor for use as antiviral drug, the MMP12 inhibitor acting specifically on extracellular MMP-12.

2. The MMP-12 inhibitor for use as antiviral drug according to claim 1, wherein the inhibitor is specific of MMP-12.

3. The MMP-12 inhibitor for use as antiviral drug according to any one of claims 1 or 2, wherein the inhibitor has low cell membrane permeability.

4. The MMP-12 inhibitor for use as antiviral drug according to any one of claims 1-3, wherein the MMP-12 inhibitor is an antibody directed against MMP-12 such as DX-2712 or a chemical compound.

5. The MMP-12 inhibitor for use as antiviral drug according to any one of claims 1-3, wherein the MMP-12 inhibitor comprises at least one, two or three negative charges at a physiological pH or is linked to a radical bearing the negative charges, directly or by a spacer.

6. The MMP-12 inhibitor for use as antiviral drug according to any one of claims 1-5, wherein the MMP-12 inhibitor is have the following formula I: 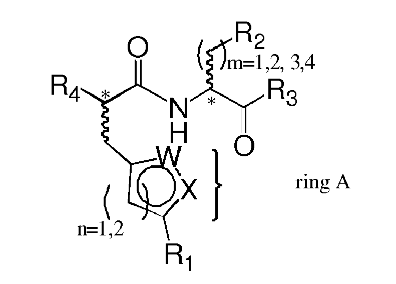 in which:
- n is 1 or 2,
- when n = 1, W and X, independently of one another, are O, N or C,
- when n = 2, W and X are C,
- R₁ is chosen from an iodine atom or a phenyl, biphenyl, 3'-chlorobiphenyl, phenoxy, phenoxymethyl, phenylethynyl, pyrimidine, 1-methyl-1 *H*-pyrazole, 5-methyl-1,2,4-oxadiazole, 1,2,3-thiadiazole, 1*H*-pyrrole, thiazole, thiophene, 3a,7a-dihydrobenzo[*d*]thiazole, 3-aminophenyl, 3-hydroxyphenyl, 3-nitrophenyl, 3-carboxyphenyl, 3-chlorophenyl, 3,5-dichlorophenyl, 3-methoxyphenyl or 3-hydroxymethylphenyl group, or a thiophene ring substituted in positions, independently of one another, 2 and/or 3 and/or 4, with a group chosen from a methyl, phenyl or 3a,7a-dihydrobenzo[*d*]thiazole group or a hydrogen atom,
- m is an integer between 1 and 4 inclusive, and
- when m = 1, R₂ is a carboxylic acid group or a 4-hydroxyphenyl group or a *1H-*imidazole group or a hydroxyl group or an isopropyl group or a methyl group,
- when m = 2, R₂ is a carboxylic acid or carboxamide group,
- when m = 3, R₂ is a carboxylic acid group,
- when m = 4, R₂ is an amino group,
- R₃ is chosen from an amino group; a carboxymethylpiperidine group, a carboxymethyl-3-aminophenyl group; a glutamate residue of L or D configuration, a homoglutamate residue, an aspartate residue, a glutamine residue, an alanine residue, a lysine residue, an arginine residue, a tyrosine residue, a histidine residue, a serine residue or a leucine residue, it being possible for the terminal carboxylic functions of said amino acids to be carboxamide functions -C(=O)NH₂, and said R₃ group being bonded to the carbonyl group of formula (1) via an amino function, and
- R₄ is selected from the group consisting of H, a carboxymethyl group - CH₂COOH and a benzylphosphinate, optionally substituted by Br in para.

7. The MMP-12 inhibitor for use as antiviral drug according to claim 6, wherein R₂ is a carboxylic acid or carboxamide group, R₃ is selected from the group consisting of a carboxymethyl-3-aminophenyl group, an aspartate residue, a glutamate residue, a glutamine residue, a lysine residue, an arginine residue and homoglutamate or their carboxamide derivative, and R₄ is selected from the group consisting of H, a carboxymethyl group and a benzylphosphinate, optionally substituted by Br in para.

8. The MMP-12 inhibitor for use as antiviral drug according to any one of claims 6-7, wherein the compound has the formula (I-A) and R₁ is a 3'-chlorobiphenyl group, R₂ is a carboxylic acid group and R₃ is selected from the group consisting of a carboxymethyl-3-aminophenyl group, an aspartate residue, a glutamate residue and homoglutamate or their carboxamide derivative, and R₄ is selected from the group consisting of H, a carboxymethyl group and a benzylphosphinate, optionally substituted by Br in para.

9. The MMP-12 inhibitor for use as antiviral drug according to any one of claims 6-7, wherein the compound has the formula (I-C) and R1 is a thiophene substituted in position 3 by phenyl, R₂ is a carboxylic acid or carboxamide group, R3 is selected from the group consisting of a carboxymethyl-3-aminophenyl group, an aspartate residue, a glutamate residue, a glutamine residue, a lysine residue, an arginine residue and homoglutamate or their carboxamide derivative, and R₄ is selected from the group consisting of H, a carboxymethyl group and a benzylphosphinate, optionally substituted by Br in para.

10. The MMP-12 inhibitor for use as antiviral drug according to any one of claims 6-7, wherein the compound has the formula (I-C) and R1 is a thiophene substituted in position 3 by phenyl, R₂ is a carboxylic acid group, R3 is selected from the group consisting of a carboxymethyl-3-aminophenyl group, an aspartate residue, a glutamate residue and homoglutamate or their carboxamide derivative, and R₄ is selected from the group consisting of H, a carboxymethyl group and a benzylphosphinate, optionally substituted by Br in para.

11. The MMP-12 inhibitor for use as antiviral drug according to any one of claims 6-7, wherein the MMP-12 inhibitor is selected from the group consisting of RXP470, and compounds 95, 95bis, 95ter, 95-4, 97, 98, 99, 101, 105 and 106, preferably selected from the group consisting of RXP470, and compounds 95, 95bis, 95ter, 97, 101 and 106.

12. The MMP-12 inhibitor for use as antiviral drug according to any one of claims 1-6, wherein the MMP-12 inhibitor is selected in the group consisting of Marimastat, GM6001, AZD1236, MMP408, AS111793, AZ11557272, and AS112108, preferably is AZD1236.

13. The MMP-12 inhibitor for use as antiviral drug according to any one of claims 1-12, wherein MMP-12 inhibitor is used in combination with an additional drug, preferably an antiviral drug.

14. The MMP-12 inhibitor for use as antiviral drug according to any one of claims 1-13, wherein it is for use for treating an acute viral infection.

15. The MMP-12 inhibitor for use as antiviral drug according to any one of claims 1-14, wherein it is for use for treating a viral infection selected from the group consisting of hepatitis B, C or D, especially chronic hepatitis C or chronic hepatitis B; herpes viruses (HSV, CMV, VZV), rotaviruses, HIV infection, laviviridae and chronic respiratory tract infections due to viral infection.
